# FASCICULE DE BREVET EUROPEEN

(11) **EP 0 689 528 B1**
(45) Date de publication et mention de la délivrance du brevet: **22.04.1998**
(21) Numéro de dépôt: 95907035.0
(22) Date de dépôt: 13.01.1995
(51) Int. Cl.: C07C 2/30

(54) **PROCEDE DE PRODUCTION D'OLEFINES ALPHA LEGERES DE PURETE AMELIOREE PAR OLIGOMERISATION DE L'ETHYLENE**
VERFAHREN ZUR HERSTELLUNG VON LEICHTEN ALPHA-OLEFINEN MIT VERBESSERTER REINHEIT DURCH OLIGOMERISIERUNG VON ETHEN
METHOD FOR PRODUCING LIGHT ALPHA OLEFINS HAVING IMPROVED PURITY BY ETHYLENE OLIGOMERISATION

(30) Priorité: 14.01.1994 FR 9400453
(43) Date de publication de la demande: 03.01.1996
(73) Titulaire: INSTITUT FRANCAIS DU PETROLE, 92506 Rueil-Malmaison Cédex (FR)
(72) Inventeur: COMMEREUC, Dominique, F-92190 Meudon (FR); GLAIZE, Yves, F-69390 Saint-Symphorien-d'Ozon (FR); HUGUES, François, F-69390 Vernaison (FR); SAUSSINE, Lucien, F-78290 Croissy-sur-Seine (FR)
(86) Numéro de dépôt international: FR9500044
(87) Numéro de publication internationale: WO9519332

(56) Documents cités:
- EP-A- 0 320 571
- EP-A- 0 578 541
- PATENT ABSTRACTS OF JAPAN vol. 15, no. 289 (C-852) (4817) 23 Juillet 1991 & JP,A,03 103 406 (IDEMITSU PETROCHEM CO) 30 Avril 1991 cité dans la demande

## Description

L'objet de la présente invention est un procédé de production d'oléfines alpha légères de pureté améliorée par oligomérisation de l'éthylène au moyen d'un catalyseur (tel que décrit dans la demande de brevet européen EP 578 541) spécifique, de type Ziegler, puis destruction dudit catalyseur par injection d'une amine suivie d'une vaporisation de l'effluent obtenu.

Plus précisément, ledit catalyseur est obtenu par mélange :
- d'un composé de zirconium de formule ZrXₓY_{y}O_{z} dans lequel X est un atome de chore ou de brome, Y est un radical choisi dans le groupe formé par les alkoxy RO⁻, les amido R₂N⁻, les carboxylates RCOO⁻, où R est un radical hydrocarbyl comprenant de 1 à 30 atomes de carbone, x et y peuvent prendre les valeurs entières de 0 à 4 et z est égal à 0 ou 0,5, la somme x + y + 2z étant égale à 4,
- avec un composé organique de formule (R₁')(R₂')C(OR₁)(OR₂) dans laquelle R₁' et R₂' sont constitués par un atome d'hydrogène ou un radical hydrocarbyl comprenant de 1 à 30 atomes de carbone, R₁ et R₂ sont des radicaux hydrocarbyl comprenant de 1 à 30 atomes de carbone,
- et avec un composé d'aluminium de formule AlR"ₙX₃₋ₙ dans laquelle R" est un radical hydrocarbyl comprenant de 1 à 6 atomes de carbone, X est un atome de chlore ou de brome, et n est un nombre compris entre 1 et 2.

Ce catalyseur est donc obtenu par mélange d'un composé de zirconium, tel que par exemple le tétrachlorure de zirconium, avec un composé organique choisi dans la classe des acétals et des cétals, résultant de la condensation d'un aldéhyde ou d'une cétone avec un monoalcool ou un polyalcool, tel que par exemple le di-(éthyl-2-hexyloxy)-2,2-propane, et avec un composé chloré ou bromé d'aluminium hydrocarbyl, tel que par exemple le sesquichlorure d'éthylaluminium.

Le procédé selon la présente invention propose une méthode spécifique de destruction du catalyseur d'oligomérisation décrit ci-dessus.

Dans une réalisation industrielle d'un procédé de catalyse homogène, en plus de la maîtrise de la réaction elle-même se pose le problème de la séparation et de l'isolement des produits à partir du mélange brut de réaction et en particulier du catalyseur qui est totalement soluble dans le mélange réactionnel.

Dans le cas de la réaction d'oligomérisation de l'éthylène, deux méthodes peuvent être mises en oeuvre dans ce but :
- élimination du catalyseur avant distillation des hydrocarbures, soit par précipitation et filtration ou centrifugation, soit par extraction biphasique par exemple par de l'eau,
- distillation directe des hydrocarbures, éthylène non converti, butène-1, hexène-1, octène-1, décène-1 et oligomères supérieurs, qui laisse en pied de distillation le catalyseur, toujours soluble mais concentré dans une fraction d'oligomères lourds, ainsi que le polymère éventuellement sous-produit.

Cependant, quelle que soit la méthode utilisée, il a été observé dans la pratique que, soit le traitement par une phase aqueuse, soit le contact prolongé entre les oligomères et le catalyseur dans les rebouilleurs des colonnes de distillation provoquent des réactions secondaires telles que des chlorations, nuisibles à la qualité des produits. ll est donc souhaitable de détruire au préalable l'activité du catalyseur, ce qui peut se faire le plus souvent par addition d'une base.

C'est ainsi que la destruction de catalyseurs d'oligomérisation de l'éthylène à base de zirconium par addition à l'issue de la réaction d'un agent anti-polymérisation et d'une amine a été décrite dans les brevets US 4 396 788, US 4 434 312, US 4 442 309 et US 4 486 615, avec pour objectif d'empêcher la formation de produits secondaires branchés, et dans le brevet EP 241 596, dans ce cas pour empêcher la formation de sous-produits chlorés. L'utilisation d'une solution aqueuse de soude ou d'ammoniaque, conjointement ou non à l'addition d'une amine, est mentionnée dans les brevets cités ci-dessus ainsi que dans les brevets EP 328 728 et JP 03 103 406.

Dans le brevet EP 320 571, le produit de la réaction d'oligomérisation est soumis à une opération de séparation de l'éthylène résiduel (appelée flashing), cette opération s'accompagne d'une précipitation du polymère. Le polymère est écrasé et le mélange obtenu est additionné d'une amine, de façon à désactiver le catalyseur. Une distillation permet de récupérer les composés oléfiniques.

La décomposition du catalyseur ternaire constitué par le mélange d'un composé de zirconium avec un acétal ou un cétal et avec un chlorure d'hydrocarbylaluminium par une phase aqueuse neutre à pH 7 fournit des oléfines alpha qui sont polluées par la présence de composés organiques chlorés. Appliquées à ce même catalyseur, les méthodes telles que décrites ci-dessus se révêlent peu efficaces, car bien qu'elles réduisent effectivement la proportion de composés chlorés dans les oligomères, celle-ci reste encore bien trop élevée pour les usages habituels de ces oléfines.

L'invention concerne ainsi un procédé de destruction et de séparation spécifique du catalyseur ternaire d'oligomérisation de l'éthylène constitué par le mélange d'un composé de zirconium avec un acétal ou un cétal et un chlorure d'hydrocarbylaluminium. Il a été trouvé que les réactions indésirables, en particulier de chloration des oléfines alpha produites, peuvent être supprimées presque totalement si l'effluent brut d'oligomérisation est soumis, dans l'ordre, aux traitements suivants, avant tout fractionnement :
1) introduction d'au moins une amine dans l'effluent du réacteur d'oligomérisation,
2) vaporisation de l'effluent traité par l'amine, soit par élévation de température, soit par abaissement de la pression, soit par action simultanée sur la température et la pression, de façon à recueillir les alpha-oléfines dans la fraction vaporisée.

Les amines utilisables selon l'invention sont de préférence les amines primaires ou secondaires de formule générale R₁R₂NH dans laquelle R₁ est l'hydrogène ou un radical hydrocarboné et R₂ est un radical hydrocarboné. R₁ et R₂ ensemble peuvent représenter un radical alcoylène. Le radical hydrocarboné peut être aliphatique, cycloaliphatique ou aromatique, et il comporte de 1 à 22 atomes de carbone.

L'ammoniaque ne convient pas, comme l'exemple comparatif ci-après le montrera.

Pour des raisons pratiques, il est souhaitable que la tension de vapeur de l'amine soit faible, de manière à ne pas contaminer de façon appréciable les oléfines alpha lors de leur distillation et/ou de leur rectification. C'est pourquoi on utilisera de préférence les amines qui comportent en tout au moins 6 atomes de carbone, par exemple de 6 à 22. Parmi les amines, on citera plus particulièrement la cyclohexylamine, l'éthyl-2-hexylamine, la laurylamine, la stéarylamine, l'oléylamine, l'aniline, la N-méthyl aniline, la dibutylamine, la didécylamine, les mélanges d'amines obtenus au départ des corps gras naturels, par exemple le suif, l'huile de palme ou l'huile de coprah.

L'amine est ajoutée de préférence à la température à laquelle a lieu la réaction d'oligomérisation c'est-à-dire entre 20 et 180 °C et de préférence entre 40 et 150 °C, ou encore à une température différente, 20 à 30 degrés en plus ou en moins par rapport à la température de réaction.

La quantité d'amine ajoutée est telle que le rapport molaire entre l'amine et le composé d'aluminium contenu dans le mélange réactionnel soit compris entre 0,1 :1 et 20:1, et de préférence entre 1 :1 et 10:1.

La vaporisation de l'effluent brut traité par l'amine peut être réalisée soit par élévation de température, soit par abaissement de la pression, soit par action simultanée sur la température et la pression. La gamme de températures et de pressions utilisables dépend de la répartition en nombre de carbone des oléfines alpha produites.

Il est souhaitable d'avoir un taux de vaporisation maximum, par exemple au moins 90 % du volume de l'effluent traité à l'amine est vaporisé, et de préférence 95 % ou plus, de manière à limiter la quantité de rejets qui devront être traités conformément aux impératifs d'environnement. Cependant ceci ne doit pas conduire à une température de vaporisation prohibitive, voire nuisible à la stabilité thermique des oléfines. On préfère respecter une température de vaporisation inférieure ou égale à 250 °C, et de préférence 200 °C.

Les produits lourds résultant de la vaporisation, et contenant le catalyseur désactivé, peuvent être incinérés, ou traités de toute manière conforme aux normes de l'environnement.

Les oligomères vaporisés sont selon les besoins dirigés vers un système de colonnes à distiller qui permet de séparer, d'une part l'éthylène non converti des oligomères, l'éthylène pouvant être renvoyé au réacteur d'oligomérisation, puis d'autre part les oligomères entre eux.

Les exemples suivants illustrent l'invention sans en limiter la portée.

### EXEMPLE 1

Dans un ballon en verre de 100 ml placé sous atmosphère inerte, on transfère à l'abri de l'humidité 2.10⁻³ mole de tétrachlorure de zirconium sublimé, puis on injecte au moyen d'une seringue hypodermique 45 ml de toluène séché et désaéré. A la suspension blanche agitée à température ambiante au moyen d'un barreau magnétique, on ajoute 2.10⁻³ mole de di-(éthyl-2-hexyloxy)-2,2-propane en solution dans 5 ml de toluène. En quelques minutes, le chlorure de zirconium se dissout et la coloration de la solution homogène ainsi obtenue évolue du jaune clair à l'orange, indiquant la formation d'un complexe.

Dans un autoclave en acier inoxydable d'un volume utile de 250 ml, muni d'une double enveloppe permettant de réguler la température par circulation d'eau ou d'huile, on introduit, dans l'ordre, sous atmosphère d'argon, et à la température ambiante, 5 ml de la solution de complexe de zirconium préparée ci-dessus, soit 0,2.10⁻³ mole de zirconium, 50 ml d'heptane, puis 1,2.10⁻³ mole de sesquichlorure d'éthylaluminium Al₂Et₃Cl₃ en solution dans 10 ml d'heptane.

La température est alors portée à 90 °C tout en introduisant dans l'autoclave de l'éthylène de manière à maintenir une pression constante de 6 MPa. Après 2 heures de réaction, l'introduction d'éthylène est arrêtée.

On injecte alors dans l'autoclave sous pression 0,55 g, soit 4,28.10⁻³ mole, d'éthyl-2-hexylamine en solution dans 3 ml de toluène, au moyen d'un sas que l'on peut porter à une pression supérieure à celle de l'autoclave. Ceci correspond à un rapport amine/Al égal à 1,78 molaire.

L'autoclave est ensuite vidé, par l'intermédiaire d'un tube interne plongeant jusqu'au fond de la cuve et d'une vanne de détente, dans un ballon en verre immergé dans un bain d'huile à 180 °C, surmonté d'une courte colonne Vigreux reliée à un ballon de recette placé dans un bain de carboglace à -78 °C. On collecte ainsi dans le ballon de recette 114 g de produit, qu'on laisse ensuite revenir à la température ambiante, la majeure partie du butène ainsi qu'un peu d'hexènes s'échappant alors en phase gazeuse. La teneur en chlore organique du produit flashé et stabilisé est inférieure à 1 partie par million en poids (ppm).

La fraction de produits lourds non flashés, qui contient aussi les résidus de catalyseur et une petite quantité de polymère, représente 3 g.

### EXEMPLE 2 (Comparatif)

On utilise dans cet exemple le même mode opératoire que dans l'exemple 1 pour préparer la solution de complexe de zirconium, pour mettre en oeuvre le catalyseur dans le même autoclave, et pour effectuer la réaction d'oligomérisation de l'éthylène dans les mêmes conditions pendant le même temps de réaction. A la fin de la réaction, on injecte dans l'autoclave de l'éthyl-2-hexylamine dans les mêmes quantités et avec la même technique que dans l'exemple 1.

On laisse alors refroidir l'autoclave, et on le dépressurise en relachant les produits gazeux dans un gazomètre. L'autoclave est ensuite ouvert et on collecte son contenu sous atmosphère inerte, ce qui représente 93 g de produit liquide stabilisé. Ce produit est lavé avec 20 ml d'une solution aqueuse de soude à 18 % en poids pour éliminer les résidus de catalyseur. La teneur en chlore organique du produit après lavage est égale à 26 ppm.

Par comparaison avec l'exemple 1, cet exemple d'une technique de l'art antérieur montre la supériorité de l'invention dans le cas du catalyseur ternaire chlorure de zirconium-acétal-sesquichlorure d'éthylaluminium.

### EXEMPLE 3 (Comparatif)

On utilise dans cet exemple le même mode opératoire que dans l'exemple 1 pour préparer la solution de complexe de zirconium, pour mettre en oeuvre le catalyseur dans le même autoclave, et pour effectuer la réaction d'oligomérisation de l'éthylène dans les mêmes conditions pendant le même temps de réaction. A la fin de la réaction, on laisse refroidir l'autoclave, et on le dépressurise en relachant les produits gazeux dans un gazomètre. L'autoclave est ensuite ouvert et on collecte son contenu sous atmosphère inerte, ce qui représente 92 g de produit liquide stabilisé.

A ce produit maintenu sous atmosphère inerte, on ajoute sous forte agitation 0,2.10⁻³ mole d'eau, c'est-à-dire un équivalent par zirconium, puis 20 ml d'une solution aqueuse d'ammoniaque à 32 % en poids. Après décantation et séparation de la phase hydrocarbonée, on détermine sa teneur en chlore organique, qui est égale à 54 ppm.

Par comparaison avec l'exemple 1, cet exemple d'une autre technique de l'art antérieur montre la supériorité de l'invention dans le cas du catalyseur ternaire chlorure de zirconium-acétal-sesquichlorure d'éthylaluminium.

### EXEMPLE 4 (Comparatif)

On utilise dans cet exemple le même mode opératoire que dans l'exemple 1 pour préparer la solution de complexe de zirconium, pour mettre en oeuvre le catalyseur dans le même autoclave, et pour effectuer la réaction d'oligomérisation de l'éthylène dans les mêmes conditions pendant le même temps de réaction. A la fin de la réaction, on injecte dans l'autoclave sous pression 3 ml d'eau au moyen d'un sas que l'on peut porter à une pression supérieure à celle de l'autoclave. On laisse refroidir l'autoclave, et on le dépressurise en relachant les produits gazeux dans un gazomètre. L'autoclave est ensuite ouvert et on collecte le produit liquide stabilisé.

Ce produit est alors distillé et chaque fraction récupérée est analysée pour sa teneur en chlore organique. On obtient ainsi 21 g d'hexènes qui contiennent 418 ppm de chlore, 12,5 g d'octènes qui contiennent 426 ppm de chlore, 7,3 g de décènes qui contiennent 553 ppm de chlore, et un pied de distillation de 10,2 g contenant 1650 ppm de chlore.

Cet exemple montre qu'avec un traitement aqueux de l'effluent brut d'oligomérisation, les sous-produits chlorés sont présents dans toutes les fractions distillées.

### EXEMPLE 5

La réaction d'oligomérisation de l'éthylène est réalisée dans une unité pilote fonctionnant en continu, et comprenant un réacteur parfaitement agité d'un volume total de 1 litre, opérant avec un contrôle de niveau à 0,7 litre liquide. Dans ce réacteur, dont la température est régulée à 120 °C au moyen d'une circulation d'huile, et dont la pression est maintenue à 6,5 MPa grâce à une vanne de détente située sur la ligne de sortie, on injecte en continu 25 g/h d'une solution de 0,7 g de chlorure de zirconium sublimé et de 0,9 g de di-(éthyl-2-hexyloxy)-2,2-propane dans 1 kg d'ortho-xylène séché et désaéré, et 25 g/h d'une solution de 17,4 g de sesquichlorure d'éthylaluminium dans 1 kg d'ortho-xylène séché et désaéré. Dans ces conditions, le débit d'éthylène à l'entrée du réacteur, asservi au contrôle de niveau, s'établit à 180 g/h.

A la sortie du réacteur, on injecte en ligne, en continu, 30 g/h d'une solution de 44,4 g de laurylamine dans 1 kg d'ortho-xylène séché et désaéré, ce qui correspond à un rapport amine/Al égal à 2,05 molaire. L'effluent traverse ensuite une colonne de flash fonctionnant à une température de 150 °C sous une pression de 0,3 MPa. La fraction flashée est envoyée à une colonne de stabilisation, et la fraction non flashée est collectée dans un pot de recette. Cette fraction non flashée, qui contient aussi les résidus de catalyseur avec un peu de polymère, représente un débit de 23 g/h. Le bilan matière autour de la colonne de stabilisation indique un débit d'oligomères flashés égal à 107 g/h.

L'analyse du chlore organique sur la fraction flashée et stabilisée donne une valeur inférieure à 1 ppm.

### EXEMPLE 6 (Comparatif)

On utilise la même unité pilote que celle décrite dans l'exemple 5 et dans les mêmes conditions, à ceci près que la colonne de flash est supprimée. L'effluent traité par la laurylamine dans les mêmes conditions que dans l'exemple 5 est donc dirigé directement vers la colonne de stabilisation.

Le produit stabilisé recueilli en pied de colonne est traité en discontinu par une solution aqueuse de soude à 18 % en poids, à raison de 2 volumes de produit pour un volume de soude.

L'analyse du chlore organique sur le produit stabilisé et traité à la soude donne une valeur de 39 ppm.

La comparaison des exemples 5 et 6 d'une opération en continu confirme l'effet bénéfique inattendu de la séquence traitement à l'amine puis flash, déjà démontré dans une opération en batch dans les exemples 1 et 2.

## Revendications

1. Procédé de conversion de l'éthylène en oléfines alpha légères dans lequel l'éthylène est mis au contact d'un catalyseur obtenu par mélange :
- d'un composé de zirconium de formule ZrXₓY_{y}O_{z} dans lequel X est un atome de chore ou de brome, Y est un radical choisi dans le groupe formé par les alkoxy RO⁻, les amido R₂N⁻, les carboxylates RCOO⁻, où R est un radical hydrocarbyl comprenant de 1 à 30 atomes de carbone, x et y peuvent prendre les valeurs entières de 0 à 4 et z est égal à 0 ou 0,5, la somme x + y + 2z étant égale à 4,
- avec un composé organique de formule (R₁')(R₂')C(OR₁)(OR₂) dans laquelle R₁' et R₂' sont constitués par un atome d'hydrogène ou un radical hydrocarbyl comprenant de 1 à 30 atomes de carbone, R₁ et R₂ sont des radicaux hydrocarbyl comprenant de 1 à 30 atomes de carbone,
- et avec un composé d'aluminium de formule AlR"ₙX₃₋ₙ dans laquelle R" est un radical hydrocarbyl comprenant de 1 à 6 atomes de carbone, X est un atome de chlore ou de brome, et n est un nombre compris entre 1 et 2,
et caractérisé en ce que pour purifier les oléfines alpha légères, dans l'effluent brut d'oligomérisation est introduite au moins une amine, et que l'effluent ainsi traité est soumis à une vaporisation de façon à recueillir les alpha-oléfines dans la fraction vaporisée.

2. Procédé selon la revendication 1, caractérisé en ce que l'amine a pour formule générale R₁R₂NH dans laquelle R₁ est l'hydrogène ou un radical hydrocarboné comportant de 1 à 22 atomes de carbone, et R₂ est un radical hydrocarboné comportant de 1 à 22 atomes de carbone.

3. Procédé selon l'une des revendications 1 ou 2, caractérisé en ce que l'amine comporte au moins 6 atomes de carbone.

4. Procédé selon l'une des revendications précédentes, caractérisé en ce que l'amine est choisie dans le groupe formé par la cyclohexylamine, l'éthyl-2-hexylamine,la laurylamine, la stéarylamine, l'oléylamine, l'aniline, la N-méthyl aniline, la dibutylamine, la didécylamine, les mélanges d'amines obtenus au départ des corps gras naturels.

5. Procédé selon l'une des revendications précédentes, caractérisé en ce que l'amine est introduite dans l'effluent brut d'oligomérisation à une température de 20 à 180 °C.

6. Procédé selon l'une des revendications précédentes, caractérisé en ce que la quantité d'amine introduite est telle que le rapport molaire entre l'amine et le composé d'aluminium est compris entre 0,1:1 et 20:1.

7. Procédé selon l'une des revendications précédentes, caractérisé en ce que la quantité d'amine introduite est telle que le rapport molaire entre l'amine et le composé d'aluminium est compris entre 1:1 et 10:1.

8. Procédé selon l'une des revendications précédentes, caractérisé en ce que la vaporisation est réalisée par élévation de température.

9. Procédé selon l'une des revendications précédentes, caractérisé en ce que la vaporisation est réalisée par abaissement de la pression.

10. Procédé selon l'une des revendications précédentes, caractérisé en ce que au moins 90 % du volume de l'effluent traité à l'amine est vaporisé.

11. Procédé selon l'une des revendications précédentes, caractérisé en ce que la tempéraure de vaporisation est inférieure ou égale à 250 °C.

## Patentansprüche

1. Verfahren zur Umwandlung von Ethylen in leichte α-Olefine, bei dem Ethylen mit einem Katalysator in Kontakt gebracht wird, der erhalten wird durch Mischen
- einer Zirkonium-Verbindung der Formel ZrXₓY_{y}O_{z}, in der X ein Chlor- oder Bromatom und Y einen Rest, ausgewählt aus der Gruppe der Alkoxyreste RO-, der Amidoreste R₂N-, der Carboxylatreste RCOO-, worin R für einen Hydrocarbylrest mit 1 bis 30 Kohlenstoffatomen steht, bedeuten und x und y ganze Zahlen zwischen 0 und 4 und z die Zahl 0 oder 0,5 sein können, wobei die Summe x + y + 2z = 4,
- mit einer organischen Verbindung der Formel (R₁')(R₂')C(OR₁)(OR₂), in der R₁' und R₂' für ein Wasserstoffatom oder einen Hydrocarbylrest mit 1 bis 30 Kohlenstoffatomen und R₁ und R₂ für Hydrocarbylreste mit 1 bis 30 Kohlenstoffatomen stehen, und
- mit einer Aluminiumverbindung der Formel AlR"ₙX₃₋ₙ, in der R" für einen Hydrocarbylrest mit 1 bis 6 Kohlenstoffatomen, X für ein Chlor- oder Bromatom und n für eine Zahl zwischen 1 und 2 stehen,
dadurch gekennzeichnet, daß zur Reinigung der leichten α-Olefine in den rohen Abstrom der Oligomerisation mindestens ein Amin eingeführt wird und daß der so behandelte Abstrom einer Verdampfung in der Weise unterworfen wird, daß die α-Olefine in der verdampften Fraktion gewonnen werden.

2. Verfahren nach Anspruch 1, dadurch gekennzeichnet, daß das Amin die allgemeine Formel R₁R₂NH hat, in der R₁ für Wasserstoff oder einen Kohlenwasserstoffrest mit 1 bis 22 Kohlenstoffatomen und R₂ für einen Kohlenwasserstoffrest mit 1 bis 22 Kohlenstoffatomen stehen.

3. Verfahren nach einem der Ansprüche 1 oder 2, dadurch gekennzeichnet, daß das Amin mindestens 6 Kohlenstoffatome aufweist.

4. Verfahren nach einem der vorhergehenden Ansprüche, dadurch gekennzeichnet, daß das Amin ausgewählt wird aus der Gruppe Cyclohexylamin, Ethyl-2-hexylamin, Laurylamin, Stearylamin, Oleylamin, Anilin, N-Methylanilin, Dibutylamin, Didecylamin und Amin-Gemischen, die aus natürlichen Fetten erhalten wurden.

5. Verfahren nach einem der vorhergehenden Ansprüche, dadurch gekennzeichnet, daß das Amin bei einer Temperatur von 20 bis 180°C in den rohen Abstrom der Oligomerisation eingeführt wird.

6. Verfahren nach einem der vorhergehenden Ansprüche, dadurch gekennzeichnet, daß die eingeführte Aminmenge derart ist, daß das Molverhältnis zwischen dem Amin und der Aluminium-Verbindung zwischen 0,1:1 und 20:1 liegt.

7. Verfahren nach einem der vorhergehenden Ansprüche, dadurch gekennzeichnet, daß die eingeführte Aminmenge derart ist, daß das Molverhältnis zwischen dem Amin und der Aluminiumverbindung zwischen 1:1 und 10:1 liegt.

8. Verfahren nach einem der vorhergehenden Ansprüche, dadurch gekennzeichnet, daß die Verdampfung durch Erhöhung der Temperatur durchgeführt wird.

9. Verfahren nach einem der vorhergehenden Ansprüche, dadurch gekennzeichnet, daß die Verdampfung durch Herabsetzung des Druckes durchgeführt wird.

10. Verfahren nach einem der vorhergehenden Ansprüche, dadurch gekennzeichnet, daß mindestens 90 Vol.-% des mit dem Amin behandelten Abstroms verdampft werden.

11. Verfahren nach einem der vorhergehenden Ansprüche, dadurch gekennzeichnet, daß die Verdampfungstemperatur ≤ 250°C ist.

## Claims

1. Method for conversion of ethylene into light alpha olefines, wherein the ethylene is placed in contact with a catalyst obtained by mixture :
- of a zirconium compound with the formula ZrXₓY_{y}O_{z} in which X is an atom of chlorine or bromide, Y is a radical selected from the group formed by RO⁻ alkoxys, R₂N⁻ aminos, and RCOO⁻ carboxylates, where R is a hydrocarbyl radical having 1 to 30 carbon atoms, x and y can have whole values from 0 to 4 and z equals 0 or 0.5, the sum of x + y + 2z being equal to 4,
- with an organic compound with the formula (R₁')(R₂')C(OR₁)(OR₂) in which R₁' and R₂' are constituted by one atom of hydrogen or a hydrocarbyl radical having 1 to 30 carbon atoms, and R₁ and R₂ are hydrocarbon radicals having 1 to 30 carbon atoms,
- and with an aluminium compound with the formula AlR"ₙX₃₋ₙ in which R" is a hydrocarbyl radical having 1 to 6 carbon atoms, X is an atom of chlorine or bromine, and n is a number between 1 and 2.
and characterised in that for purifying light alpha olefines, at least one amine is introduced into the raw oligomerisation effluent, and that the effluent thus treated is subjected to vapourisation so that the alpha olefines are collected in the vapourised fraction.

2. Method according to claim 1, characterised in that the amine has the general formula R₁R₂NH, in which R₁ is hydrogen or a hydrocarbonic radical having 1 to 22 carbon atoms, and R₂ is a hydrocarbonic radical having 1 to 22 carbon atoms.

3. Method according to one of claims 1 or 2, characterised in that the amine has at least 6 carbon atoms.

4. Method according to one of the preceding claims, characterised in that the amine is selected from the group formed by cyclohexylamine, ethyl-2-hexylamine, laurylamine, stearylamine, oleylamine, aniline, N-methyl aniline, dibutylamine, didecylamine, and mixtures obtained from natural fatty substances.

5. Method according to one of the preceding claims, characterised in that the amine is introduced into the raw oligomerisation effluent at a temperature of 20 to 180°C.

6. Method according to one of the preceding claims, characterised in that the quantity of amine introduced is such that the molar ratio between the amine and the aluminium compound is between 0.1:1 and 20:1.

7. Method according to one of the preceding claims, characterised in that the quantity of amine introduced is such that the molar ratio between the amine and the aluminium compound is between 1:1 and 10:1.

8. Method according to one of the preceding claims, characterised in that the vapourisation is carried out by increasing the temperature.

9. Method according to one of the preceding claims, characterised in that the vapourisation is carried out by lowering the pressure.

10. Method according to one of the preceding claims, characterised in that at least 90% of the volume of the effluent treated with amine is vapourised.

11. Method according to one of the preceding claims, characterised in that the vapourisation temperature is less than or equal to 250°C.
